# EUROPÄISCHE PATENTSCHRIFT

(11) **EP 1 132 395 B1**
(45) Veröffentlichungstag und Bekanntmachung des Hinweises auf die Patenterteilung: **29.10.2003**
(21) Anmeldenummer: 01201272.0
(22) Anmeldetag: 07.07.1998
(51) Int. Cl.: C07K 5/08, C07K 5/06

(54) **Verfahren zur Herstellung eines pharmazeutisch aktiven Peptides**
Process for the preparation of a pharmacologically active peptide
Procédé pour la préparation d'un peptide pharmacologiquement actif

(30) Priorität: 07.07.1997 CH 165197
(43) Veröffentlichungstag der Anmeldung: 12.09.2001
(62) Teilanmeldung aus: 98929194.3
(73) Patentinhaber: PTC Pharma AG, 3000 Bern 25 (CH)
(72) Erfinder: Mehlem, Francesco, 3048 Worblaufen (CH)
(74) Vertreter: Liebetanz, Michael, Dipl.-Phys.

(56) Entgegenhaltungen:
- DE-A- 2 128 549

## Beschreibung

Die vorliegende Erfindung bezieht sich auf ein Verfahren zur Herstellung eines pharmazeutisch aktiven Peptides, das L-m-Sarcolysin als Aminosäurebaustein enthält. Der Wirkstoff dient insbesondere zur Chemotherapie gegen Krebsleiden und wird speziell gegen Melanome eingesetzt. Eine Trägersubstanz auf Basis von Cyclodextrin dient einer verzögerten Freisetzung des Wirkstoffes und ist für eine genügende Bioverfügbarkeit während einer ausreichend langen Zeitdauer verantwortlich.

Ein Komplex von sechs Peptiden, die m-L-Sarcolysin enthalten, ist unter dem Warennamen "Peptichemio" (Istituto Sieroterapico Milanese S. Belfanti, Milano, IT) für die Chemotherapie gegen Krebs bekannt geworden. Es wurde gefunden, dass die Aktivität der einzelnen Peptide verschieden ist und dass besonders ein Vertreter eine sehr hohe Toxizität für Melanomzellen aufweist. Die Peptide sind eine Entwicklung, welche mit dem Produkt "Melphalan", d.h. 4-[bis(2-Chlorethyl)]-amino-L-phenylalanin begonnen hat Es wurde gefunden, dass dieses Produkt eine zytostatische Wirkung hat und sowohl für die Myelom- als auch für die Melanomtherapie eingesetzt werden kann. Zur Weiterentwicklung des Wirkstoffes wurden Derivate des Produktes hergestellt. Daraus resultierte auch das L-m-Sarcolysin, das weiter deriviert wurde, indem Peptide hergestellt wurden, welche die modifizierte Aminosäure als Baustein enthielten. Eine Kombination von 6 derartigen Oligopeptiden bildete das aktive Prinzip der Antitumormittel "Peptichemio". Die 6 Peptide sind folgende:
- L-Seryl-L-p-fluorphenylalanyl-L-m-sarcolysyl-ethylester
- L-Prolyl-L-m-sarcolysyl-L-p-fluorphenylalanin-ethylester
- L-m-Sarcolysyl-N-nitro-L-arginyl-L-norvalin-ethylester
- L-p-Fluorphenylalanyl-L-m-sarcolysyl-L-asparagin-ethylester
- L-p-Fluorphenylalanyl-glycyl-L-m-sarcolysyl-norvalin-ethylester
- L-m-Sarcolysyl-L-arginyl-L-lysyl-L-m-sarcolysyl-L-histidinmethylester

Die Peptide liegen vorzugsweise in Form von Hydrochloriden oder Hydrobromiden vor.

Es wurde gefunden, dass Peptichemio weniger toxisch gegen humane Lymphoplasten ist als m-L-Sarcolysin allein. Zusätzlich zur kleineren Toxizität von Peptichemio wurde eine verminderte Bildung von DNA-Vemetzungen festgestellt. Im Gegensatz dazu wurde eine erhöhte Zytotoxizität von Peptichemio gegen humane Melanomzellinien festgestellt, im Vergleich zum m-L-Sarcolysin. Hier war die höhere Zytotoxizität mit einer grösseren DNA-Vernetzung verbunden. Die Vergleichsanalyse der 6 Peptide zeigte Unterschiede in der Zytotoxizität gegen Melanomzellen. Eines der 6 Peptide zeigte eine beträchtlich höhere Zytotoxizität im Vergleich zum Peptichemio selbst (R. Levenson, et al., Radiumhemmet, Karolinska Hospital, Stockholm SE, Eur. J. Cancer Clin. Oncol.; 23: 6, 783-788, 1987). Gemäss diesen Studien wurde gefunden, dass das Peptid L-Propyl-m-sarcolysyl-L-p-fluorphenylalanin (PSF) 35 × bzw. 28 × toxischer gegen RPMI 8322 Melanomzellen war als Melphalan bzw. m-Sarcolysin. Ähnliche Unterschiede zwischen den Wirkstoffen wurden auch für andere Melanomzellinien gefunden.

Damit die in vitro gefundene Aktivität ebenfalls in vivo entfaltet werden kann, muss die Bioverfügbarkeit des Wirkstoffes im Körper ausreichend sein. Es muss eine genügend hohe Konzentration während einer genügenden Zeitdauer vorhanden sein bzw. die Halbwertszeit des aktiven Prinzips muss genügend sein.

Aus DE 2128549 A ist ein Verfahren zur Herstellung von L-Prolyl-Lm-sarcolysyl-L-p-fluorphenylalanin-ethylester bekannt, wo das Molekül sequentiell vom N-Terminus her aufgebaut wird. Das Verfahren erfordert in einer Stufe einen Chromatographieschritt zu Reinigung eines Zwischenproduktes, das als Öl anfällt. Ein Nachteil dieses Verfahrens ist auch die verhältnismässig niedrige Ausbeute des Endprodukts bezogen auf das Ausgangsmaterial m-Phenylalanin-ethylester.

Es ist demzufolge Aufgabe der vorliegenden Erfindung, ein verbessertes Verfahren zur Herstellung des bevorzugten Wirkstoffes zur Verfügung zu stellen.

L-Prolyl-m-sarcolysyl-L-p-fluorphenylalanin (PSF) ist der bevorzugte Wirkstoff, Zur Regulierung der Bioverfügbarkeit wird er mit einem Cyclodextrinträgerstoff, vorzugsweise Hydroxypropyl- β-cyclodextrin formuliert. (Pitha et al. in "Hydroxypropyl-β-cyclodextrin preparation and characterization", International Journal of Pharmaceutics, 29: 73 bis 83 (1986)). Zur parenteralen Verabreichung wird ein substitutiertes β-Cyclodextrin, vorzugsweise Hydroxypropyl-β-cyclodextrin verwendet. Die Cyclodextrinverbindung bildet mit den Peptiden einen Komplex und bewirkt bei parenteraler Applikation, dass der Wirkstoff im Organismus in genügender Konzentration zur Verfügung gestellt wird. Vorzugsweise wird als Wirkstoff PSF zusammen mit Hydroxpropyl-β-Cyclodextrin verwendet, wobei ein Mol-Verhältnis PSF zu Hydroxpropyl-β-Cyclodextrin im Bereich von 1:1 bis 1:10 verwendet wird. Typische Beispiele derartiger Verhältnisse sind 1:1, 1:2, 1:3. Der Wirkstoff bildet mit der Cyclodextrinverbindung einen Komplex, in welchem das Peptid stabilisiert wird. Die Halbwertszeiten in der Grössenordnung von 10 bis 30 min. des Wirkstoffes in einer Körperflüssigkeit werden durch Inklusion in einen Komplex mit Cyclodextrin erhöht, wobei eine Steuerung durch das obenerwähnte Verhältnis möglich ist. Der Komplex kann durch den Organismus besser aufgenommen und absorbiert werden, wobei die Bioverfügbarkeit positiv beeinflusst wird. Das Verhältnis muss der Krankheit des Patienten und seinem Gesundheitszustand angepasst werden. Das Produkt kann als festes Produkt oder als Konzentrat vorgelegt werden, welches zur Herstellung von injizierbaren Lösungen oder Infusionen dient. Falls therapeutisch erforderlich, kann die pharmazeutische Formulierung auch andere Wirkstoffe wie z.B. Chlorphenamin enthalten.

Für orale Formulierungen wird das PSF mit α-, oder -β- oder γ-Cyclodextrin vermischt. Das Verhältnis von Peptid zu Cyclodextrin beträgt hier beispielsweise 1 : 1 bis 1 :10, typischerweise 1:1, 1:2, 1:3. Die Kombination wird gegebenenfalls zusammen mit einem geeigneten Trägerstoff in Kapseln verpackt.

### Beispiel

### Synthese von L-prolyl-L-m-sarcolysyl-L-D-fluorphenylalanin-ethylesterhydrochlorid

### a) N-Carbobenzoxy-L-m-sarcolysyl-L-p-fluorphenylalanin-ethylester

52,5 g L-p-Fluorphenylalaninethylester Hydrochlorid werden mit 75 ml Na₂CO₃ (Natriumcarbonat) gesättigte Lösung und 150 ml CHCl₃ behandelt. Die Mischung wird ausgeschüttelt und die organische Phase wird getrennt und aufbewahrt. Die wässrige Phase wird mit 75 ml CHCl₃ ein zweites Mal ausgeschüttelt. Die vereinigten Chloroformextrakte werden gemischt und einmal mit Wasser gewaschen, und dann von der wässrigen Phase getrennt und auf wasserfreiem Na₂SO₄ getrocknet Die Konzentration von Aminosäureester wird durch eine Titration mit HClO₄ (Perchlorsäure) bestimmt. Die Ausbeute entspricht ungefähr dem theoretischen Wert; sie liegt bei 98%.

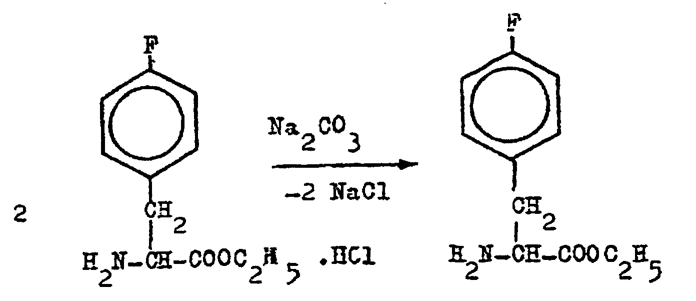

286,5 ml einer Chloroformlösung, die 0,1905 Mol L-p-Fluorphenylalaninethylester enthält, werden mit 83,7 g (0,1905 Mole) N-Cbzo-Lm-sarcolysin versetzt. Die Lösung wird auf einem Eisbad gekühlt.

Der gekühlten Lösung werden unter Rühren 41,25 g (0,200 Mol Dicyclohexylcarbodiimid - DCC) und 60 ml Chloroform dazugegeben, wobei die Lösung während 30 min. unter gleichzeitiger Kühlung ständig gerührt wird. Unter Umständen kann die Mischung zu fester Masse erstarren. In diesem Fall wird die Masse durch Zugabe von 150 ml Chloroform wieder flüssig gemacht, wobei sie unter leichtem Erwärmen gerührt wird. Auf diese Weise wird die Auflösung des ausgefallenen Produktes beschleunigt. Die Reaktion ist 2 h nach Zugabe des DDC beendet. Das Reaktionsende wird durch TLC-Kontrolle festgestellt (Dünnschochtchromatographie; Kielgel G-Schicht, Lösungsmittel: Chloroform + Aceton 9:1, Sichtbarmachung durch Besprühen mit verdünnter, saurer KMnO₄-Lösung). Der ausgefallene Dicyclohexylharnstoff wird durch Filtration abgetrennt. Die Lösung wird zuerst mit wenig Wasser, dann mit gesättigter Na₂CO₃-Lösung gewaschen. Die Chloroformlösung wird noch einmal mit Wasser ausgeschüttelt und dann mit Na₂SO₄ getrocknet. Das Lösungsmittel wird unter Vakuum verdampft und entfernt. Nach Trocknung werden 140,25 g leicht gelblich gefärbtes Produkt erhalten (Ausbeute 98,3%).
Die gewonnene Substanz hat einen Schmelzpunkt von 123-124,5°C und ist chromatographisch homogen. Durch Kristallisation von 4,5 g Substanz aus 37,5 ml Ethylalkohol werden 3,75 g helleres Produkt gewonnen mit einem Schmelzpunkt von 125-126 °C. α_{D}²⁰: 27.7 (c = 2, CHCl₃). 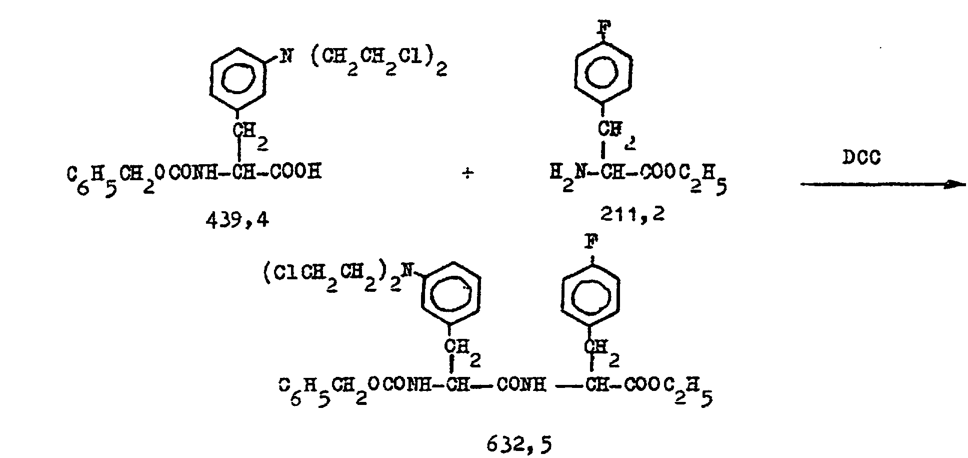
Analyse für C₃₂H₃₆Cl₂FN₃O₅
N% = 6,67 (berechnet 6,66
Cl% = 11,5 (berechnet = 11,2)

### b) L-m-Sarcolysyl-L-p-fluorphenylalanin-ethylester

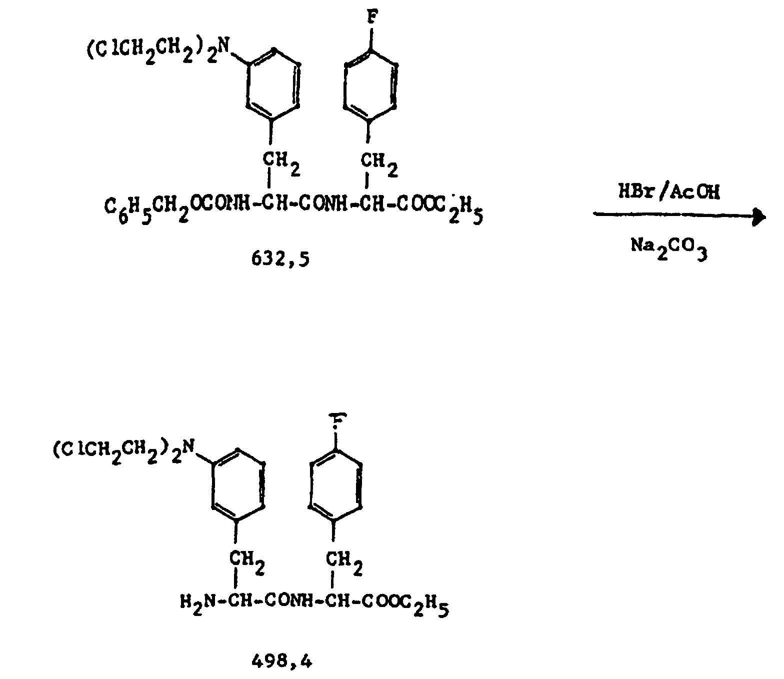

Unter Ausschluss der Luftfeuchtigkeit werden zu 390 g (0, 616 mol) die N-Carbobenzoxy-L-m-sarcolysyl-L-p-fluorphenylalanin-ethylester unter langsamem Rühren 600 ml HBr in Eisessig (33 %) zugegeben. Die Auflösung und das Aufhören der CO₂-Entwicklung findet nach 40 Minuten statt. Es wird während weiteren 20 Minuten unter Rühren stehengelassen und mit ca. 400 ml Ether verdünnt. Man giesst das gesamte in 5 Ether, welcher unter ständigem Rühren gehalten wird, dekantiert und wäscht das ausgefallene Oel 2 x mit 2 I Ether unter Dekantieren. Das Oel wird unter Rühren mit 4 Wasser behandelt und man erhält einen Feststoff, welcher nach ca. 30 min. durch Filtration gesammelt wird und vollständig mit insgesamt 1500 ml Wasser und 500 ml Ether gewaschen wird. Das so erhaltene Bromhydrat wird in 2 Ethylacetat suspendiert und unter Rühren mit 450 ml gesättigter Natriumcarbonatlösung behandelt, derart bis die Lösung alkalisch ist. Nachdem die Auflösung stattgefunden hat, filtriert man auf der Nutsche, um den supendierten Dicylohexylharnstoff (sehr wenig) zu entfernen. In einem Scheidetrichter trennt man die organische Schicht von der wässerigen Phase ab, und die wässrige Phase wird mit weiteren 500 ml Ethylacetat extrahiert. Die gereinigten Extrakte werden mit 300 ml Wasser gewaschen, Na₂CO₄ getrocknet und mit Norit behandelt. Es wird filtriert und das Filtrat wird unter dem Vakuum getrocknet (40°C). Der Rückstand wird noch vor seiner Festigung in 500 bis 1000 ml Ether aufgenommen. Aus der erhaltenen Lösung wird während der Nacht ein weisses Produkt ausgefällt. Ausbeute: 247 g (80,4 %)
Smp. 100 -102 °C.
α_{D}²⁰ = -7,5° (c=2, Chloroform)
TLC (BuOH/AcOH/H₂O 65:15:25; KMnO₄ verdünnt):
Eine Bande, Rf = 0,74
Analyse für C₂₄H₃₀Cl₂FN₃O₃
N% = 8,34 (berechnet 8,43)
Cl% = 14,1 (berechnet 14,2)

### c) N-Carbobenzoxy-L-prolyl-L-m-sarcolysyl-L-p-fluorphenylalaninethylester

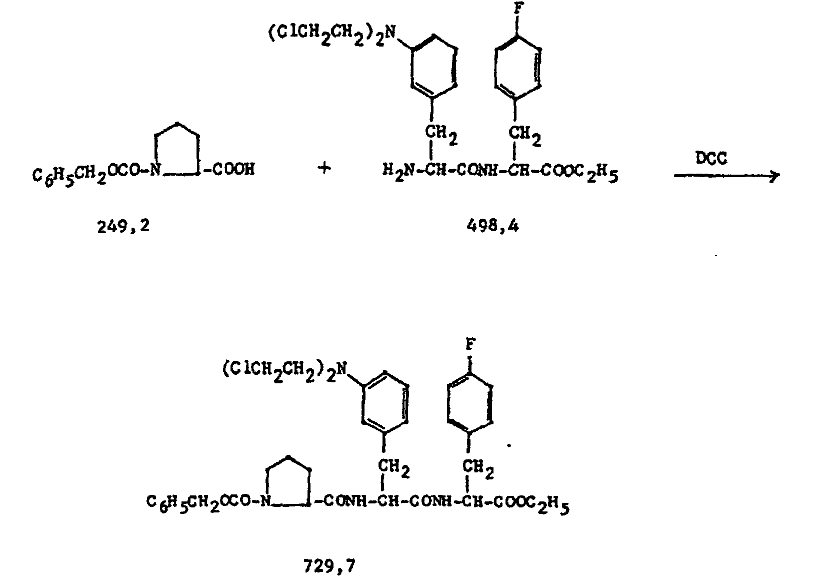

Eine Mischung von 249 g (0,5 mol) L-m-sarcolysyl-L-pfluorphenylalaninethylester, 125 g (0,5 mol) N-Cbzo-L-Prolin und 109 g (0,525 mol) DCC in 3000 ml Chloroform wird während 30 Minuten unter Rühren stehen gelassen, mit externer Kühlung während weiteren 90 Minuten bei Zimmertemperatur (TLC, Silikagel G, Chf/Me₂CO 9:1; oder mit BuOH/AcOH/H₂O 65:15:25; KMnO₄, verdünnt, sauer). Nach der Entfernung des Dicyclohexylharnstoff durch Filtration wird das Lösungsmittel unter Vakuum abgedampft und der Rückstand wird noch in flüssigem Zustand in 800 ml Ether gegossen. Von der erhaltenen Lösung fällt langsam das Produkt aus, welches auf einem Filter gesammelt wird. Ausbeute 290 g (78,5%).
Smp. = 148-150°C, α_{D}²⁰ = -42,4° (c=2; Chloroform)
Analyse für C₃₇H₄₃FCl₂N₄O₆
N% = 7,78% (berechnet 7,68)
Cl% = 9,6 (berechnet 9,7)

### d) L-Prolyl-L-m-sarcolysyl-L-p-fluorphenylalanin-ethylester-hydrochlorid

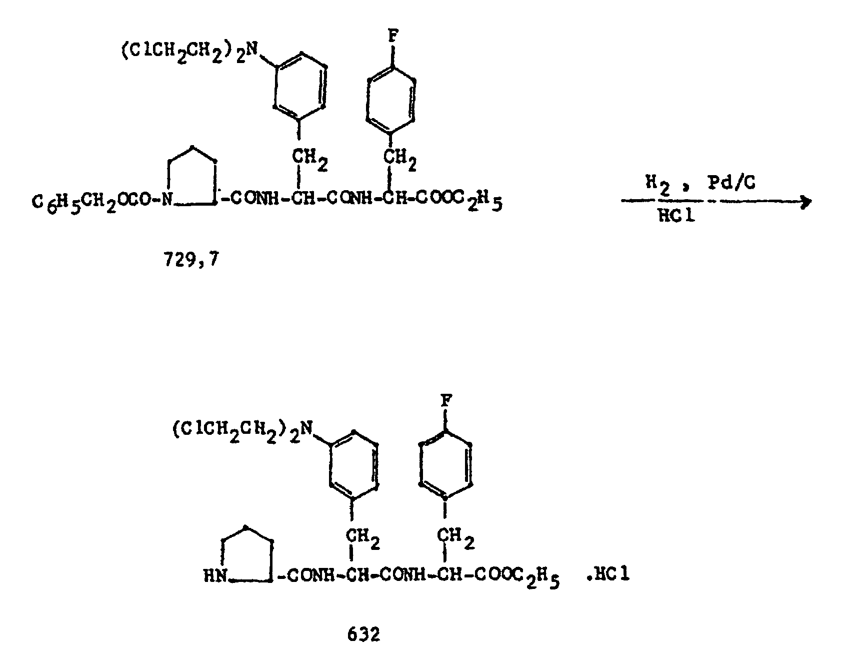

Eine Mischung von 157,5 (0,261 mol) N-Carbobenzoxy-L-prolyl-L-msarcolysyl-L-p-fluorphenylalanin-ethylester und 30 g Palladium auf Kohlenstoff 5% wird suspendiert unter einem Stickstoffstrom in 15 ml Eisessig und 1750 ml Methanol. Die Reaktionsmischung wird unter Rühren gehalten und wird unter einem Wasserstoffstrom reduziert. Nach der Beendigung der CO₂-Entwicklung (nach 4 -5 Stunden) wird eine TLC-Chromatographiekontrolle durchgeführt (Kieselgel G), wobei mit Chloroform-Aceton 9:1 eluiert wird und mit verdünntem KMnO₄ sichtbar gemacht wird.

Nachdem der Entfernung des Katalysators durch Filtration wird das Filtrat mit konzentrierter ethanolischer HCI in stöchiometrischer Menge oder wenig mehr angesäuert. Der weisse, kristalline Niederschlag, welcher sich langsam bildet, wird auf einem Filter gesammelt und mit Ethanol oder mit Ether gewaschen: 85 g. Das Filtrat wird praktisch bis zur Trockenheit konzentriert und der Rückstand wird aus Ethanol umkristallisiert: 25 g. Vollständige Ausbeute: 110 g (80, 5%); Smp. 122 - 124 °C (Änderung des Aggregatszustandes)
α_{D}²⁰ = ⁻13,0° ± 0,5 (c= 2; MeOH)
TLC (Kieselgel G; BuOH/AcOH/H₂O 65:15:25; KMnO₄ verdünnt: eine Bande Rf = 0,54.
Analyse für C₂₉H₃₈Cl₃FN₄O4
N % = 8,93% (berechnet 8,86)
Cl % = 16,7 % (berechnet 16,8)
Cl-% = 5,65% (berechnet 5,6)

## Patentansprüche

1. Verfahren zur Herstellung von L-Prolyl-L-m-sarcolysyl-L-p-fluorphenylalanin-ethylester, **gekennzeichnet durch** die Stufen bis e) entsprechend folgenden Reaktionsgleichungen.

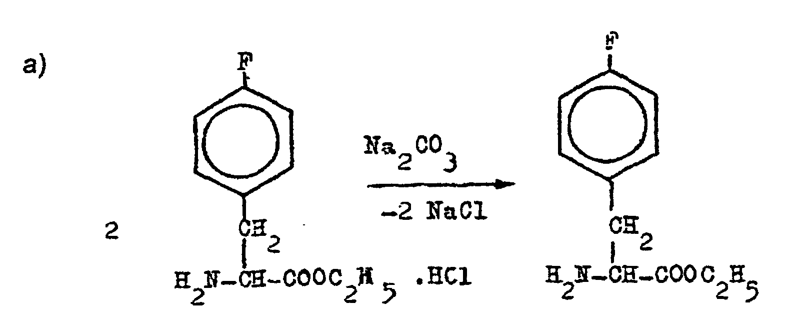

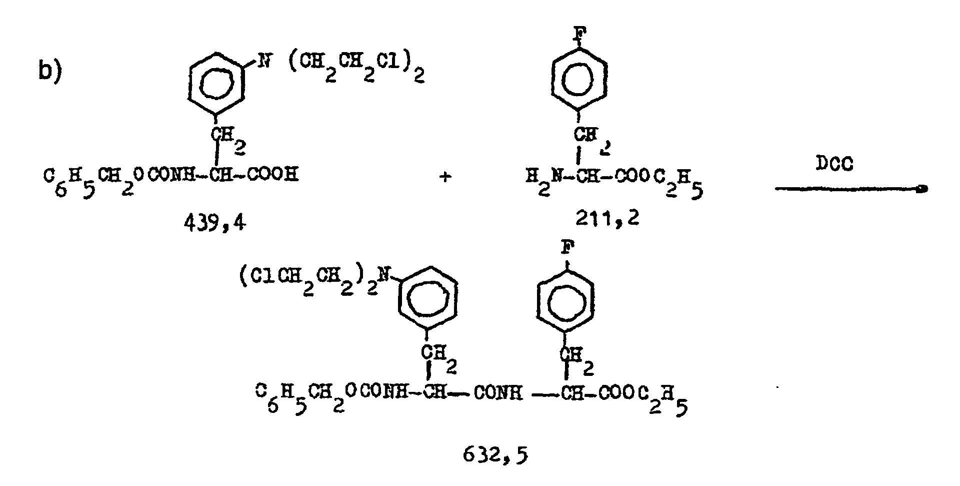

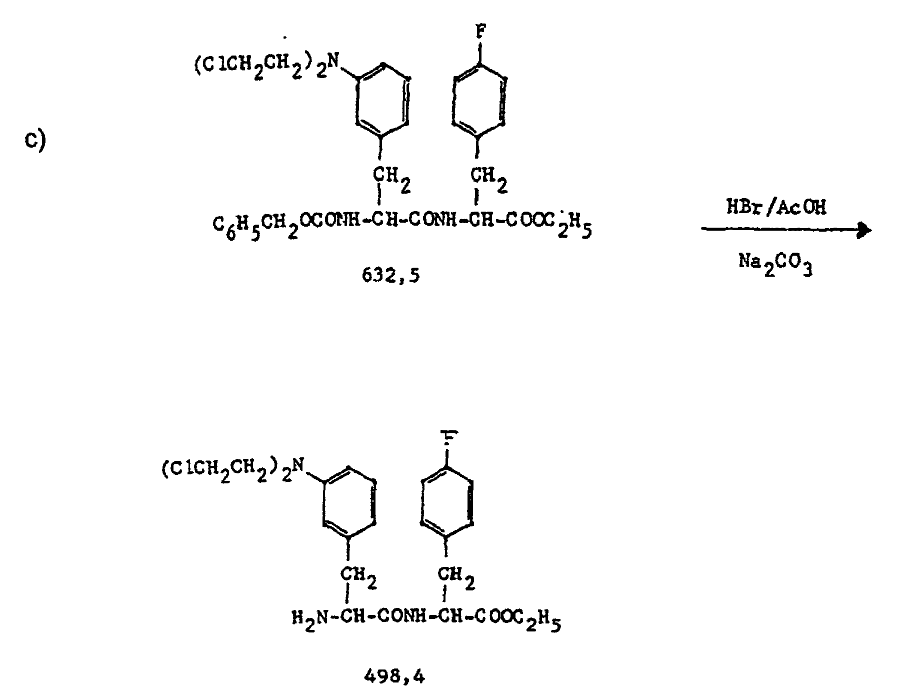

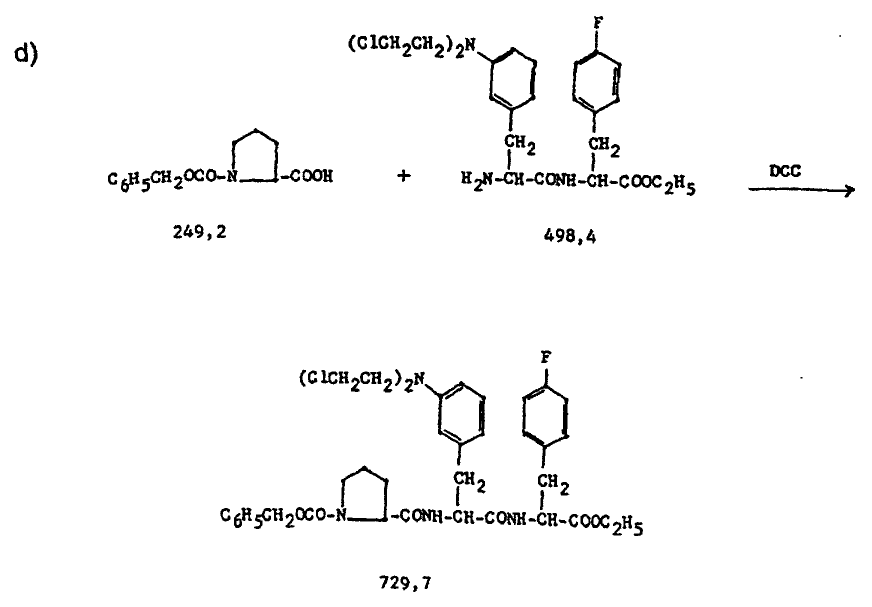

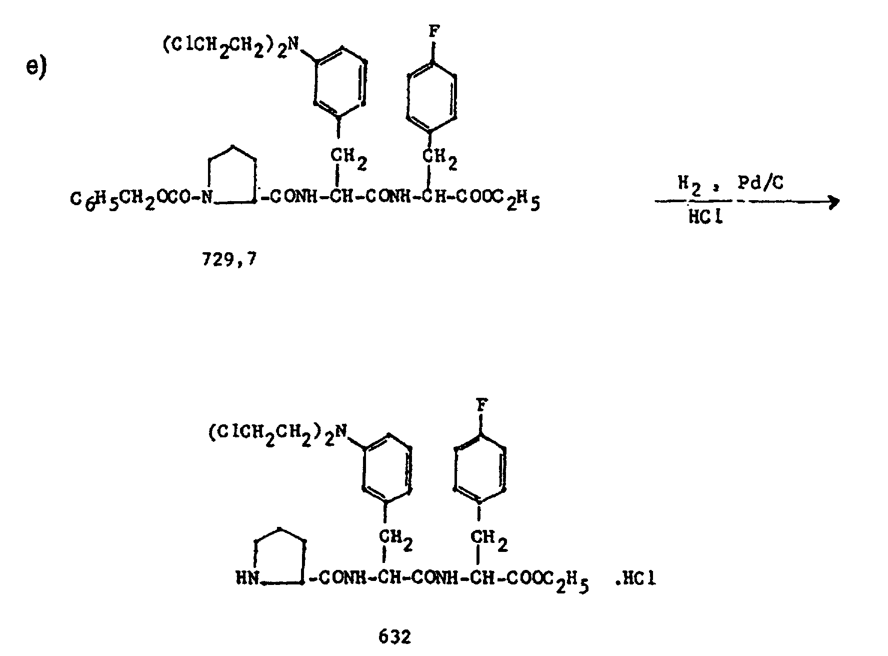

## Claims

1. Process for the preparation of L-prolyl-L-m-sarcolysyl-L-p-fluorphenylalanin-ethyl ester **characterized by** the steps a) to e) according to the following reaction equations:

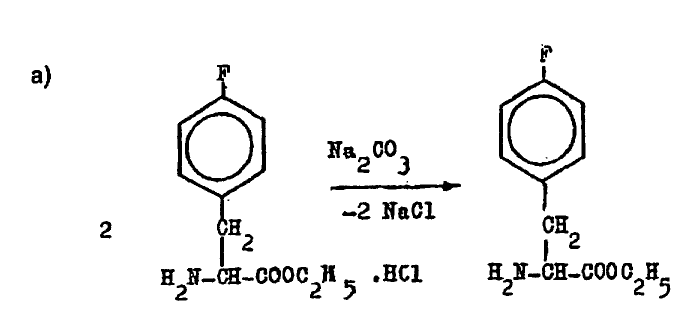

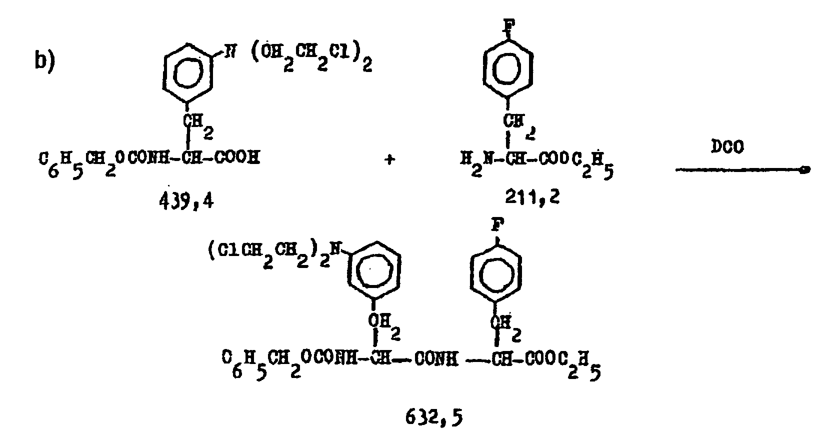

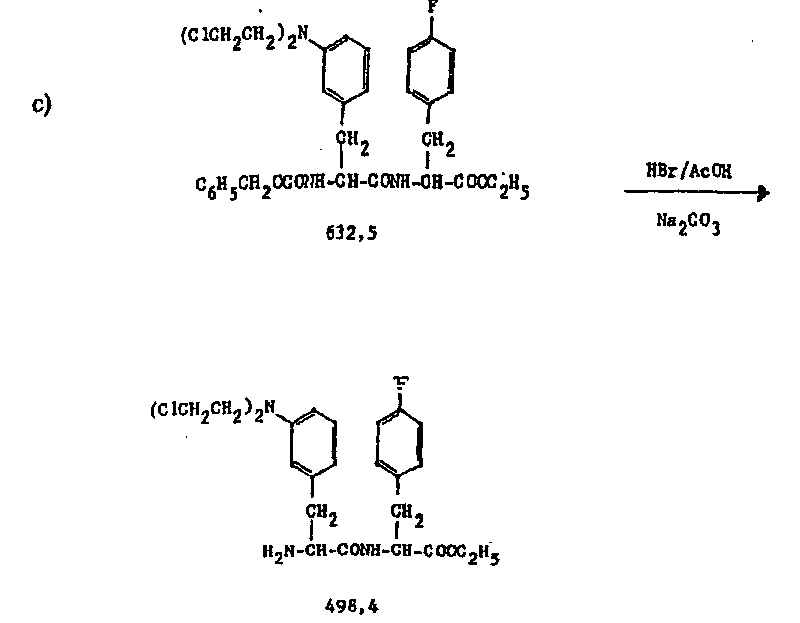

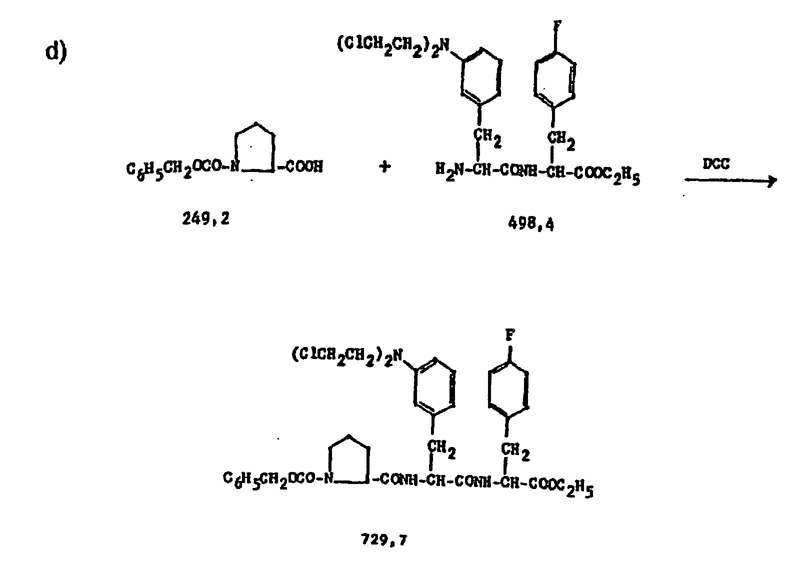

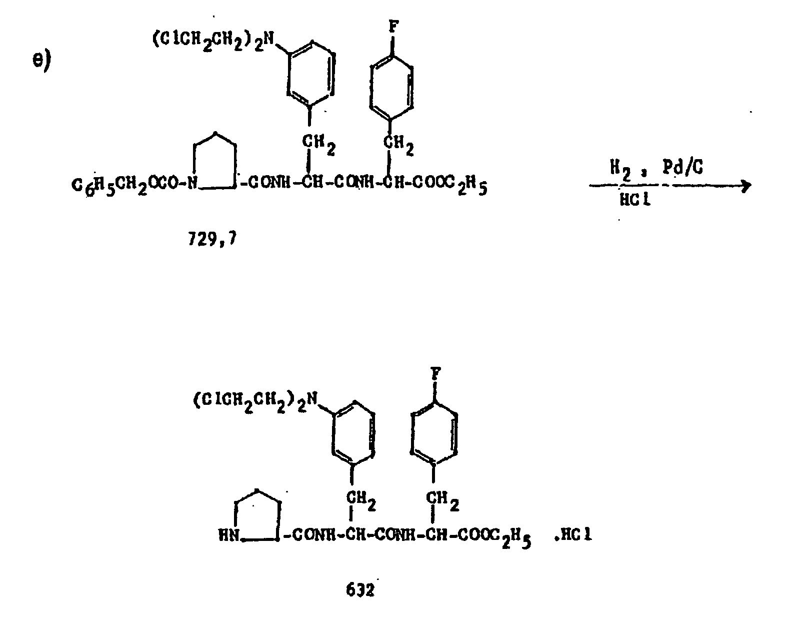

## Revendications

1. Procédé pour la préparation de L-prolyl-L-m-sarcolysyl-L-p-fluorphenylalanin-éthyle ester **caractérisé par** les étapes a) à e) selon les réactions suivantes:
